# EUROPEAN PATENT APPLICATION

(11) **EP 4 686 404 A1**
(43) Date of publication of application: **04.02.2026**
(21) Application number: 24192471.1
(22) Date of filing: 01.08.2024
(51) Int. Cl.: A01N 1/125

(54) **CELL PRESERVATION SOLUTION COMPOSITION AND CELL PRESERVATION METHOD**

(71) Applicant: Accomodate Preserve Co.,Ltd., Datong, Taipei City 103022 (TW)
(72) Inventor: Huang, Hung-Jui, 80742 Kaohsiung City (TW)
(74) Representative: Lang, Christian

(57) **Abstract**

A cell preservation solution composition, excluding dimethyl sulfoxide (DMSO), includes a copolymer of compound A and compound B. In a composition of 100 wt% of the cell preservation solution composition, the compound A ranges from 60.00wt% to 99.95wt%, and compound B ranges from 0.05 wt% to 40.00 wt%.

## Description

### FIELD OF THE INVENTION

The present invention relates to a cell preservation solution composition, particularly relates to a cell preservation solution composition and a cell preservation method for hypothermic preservation of target objects.

### BACKGROUND OF THE INVENTION

Cryopreservation technology at a temperature of 0°C or below is usually used for long-term preservation of biological materials, such as cells and tissues of animals (including human cells and tissues) and plants. Efficient long-term storage of these mammalian cells is critical to the successful use of these cells both clinically and as research tools. For example, stem cells can be used in cell transplantation and tissue engineering regenerative medicine. Cryopreserved oocytes, sperm and embryos can be used in assisted reproductive technologies. In addition, in transplant medicine, living tissues such as skin, corneas, pancreatic islets, and heart valves need to be cryopreserved.

The present technology has pointed out that during the freezing process, the formation of intracellular ice crystals and the imbalance of osmotic pressure may damage cells. In order to avoid the above situation, cryoprotectant (CPA) are used to freeze cells. It is used to protect the vitality of cells and tissues. The cryoprotectant is characterized by three main chemical classes: polyols such as glycols, glycerol, amides, and styrene. Commonly used cryoprotectant include glycerin, dimethyl sulfoxide (DMSO, Dimethyl sulfoxide) and polyethylene glycol (PEG, polyethylene glycol). In addition, some additives, such as polymer materials and sugars, can be added to further reduce the damage to cells and tissues during cryopreservation.

Among these cryoprotectants, DMSO is highly cell permeable and is the most effective and frequently used cryoprotectant. However, DMSO is physiologically toxic and is currently known to cause hypertension, nausea, and vomiting when DMSO-treated cells are infused into recipients. In addition, after the thawed cells are cultured or injected into the recipient, the toxicity of DMSO can easily reduce the survival rate and/or function of the cells.

Slow freezing and vitrification are two traditional methods for freezing and preserving biological materials. During slow freezing, cells are cooled to a temperature slightly below the equilibrium freezing point and ice nuclei form in the medium outside the cells. As ice crystals form in the extracellular solution, the concentration of external solutes gradually increases. The cells thus become dehydrated and the melting point of the cytoplasm decreases. Avoids the formation of intracellular ice crystals. The definition of vitrification is when the consistency of the sample reaches a certain level, and it becomes amorphous and fixed. If the temperature is rapidly cooled, a vitrified state can be induced in most liquids, and the addition of cryoprotectants can reduce the need for high cooling rates.

In addition, the step of preserving the target object in the prior art includes the target object is first separated from the blood, the target object such as peripheral blood mononuclear cells (PMBC, peripheral blood mononuclear cells), stem cells (stem cells) or the stem cells is separated from the tissue. Next, human NK cells, immune cells, and stem cells are performed with expansion culture process. The culture medium is removed from the expansion cultured human NK cells, immune cells, and stem cells, and then perform a cell washing process. Next, the washed human NK cells, immune cells, and stem cells are resuspended in the preservation solution so that the cell concentration is 1×10⁵-1×10⁹ cell/ml, and the cells is preserved at a preservation equipment with a preservation temperature of 2°C-8°C. According to the above, the preservation solution must be stored in a preservation manner, and the preservation temperature is 2°C-8°C. Because the preservation solution needs to be kept under hypothermic preservation conditions when replacing the culture medium, the preservation solution must be added while maintaining low temperature (i.e., hypothermic preservation temperature) after the cell expansion culture, removing the culture medium, and washing the cells. However, the current products on the market have poor preservation effects, poor effective preservation time and poor cell survival rate, and cannot effectively preserve cells. In addition, the specimen has a scarcity that cannot be reacquired or reproduced. If the cell preparation product is autologous, it also has a scarcity that cannot be reacquired or reproduced. The cell preparation product may be allogeneic and has difficulty in mass production. If the preservation efficiency of the preservation solution itself is poor and it can easily lead to misleading result. By then, it will have lost its activity during the end-of-use inspection, which will cause a waste of medical resources.

### SUMMARY OF THE INVENTION

According to the drawbacks of the conventional prior art, the present invention provides a cell preservation solution composition, which does not need to store in a specific preservation temperature range, such as 2°C-8°C. The applicable preservation temperature ranges from hypothermic preservation temperature to room temperature.

It is another object of the present invention is to provide a cell preservation solution composition. The cell preservation solution composition does not to be specifically hypothermic preservation. After the cell(s) is performed with an expansion culture process, removing the culture medium, and washing the cell(s), the cell preservation solution composition of the present invention can add directly into the above cell(s), so that there is no need for pre-cooling or maintaining at a low temperature environment, thereby, the cell preservation solution composition of the present invention can effectively expand the storage time and improve the cell survival rate for effective cell preservation.

According to above objects, the present invention provides a cell preservation solution composition, excluding dimethyl sulfoxide (DMSO), and the cell preservation solution composition includes a copolymer containing compound A and compound B, in composition of 100% of the cell preservation solution composition, the compound A ranges from 60.00wt% to 99.5wt% and the compound B ranges from 0.05wt% to 40.00wt%.

According to above objects, the present invention further provides a method for hypothermic preservation of cell, the steps of method include providing a cell or a plurality of cells have been performed with an expansion culture process; performing a centrifugation process to remove a supernatant of the cell or the plurality of cells; mixing the cell or the plurality of cells with a cell preservation solution composition to obtain a first mixed solution; performing a hypothermic preservation process to the first mixed solution until a desired preservation time; and mixing the first mixed solution that has reached the desired preservation time with a culture medium to obtain a second mixed solution, in which the cell preservation solution composition includes compound A and compound B, in composition of 100% of the cell preservation solution composition, the compound A ranges from 60.00wt% to 99.5wt% and the compound B ranges from 0.05wt% to 40.00wt%.

According to above objects, the present invention also provides a method for hypothermic preservation of cell, the steps of method include providing a cell or a plurality of cells is taken from a cell bank; performing a performing a centrifugation process to remove a supernatant of the cell or the plurality of cells; mixing the cell or the plurality of cells after removing the supernatant with a cell preservation solution composition to obtain a first mixed solution; performing a hypothermic preservation process on the first mixture until a desired preservation time; and mixing the first mixed solution that has reached the desired preservation time with a culture medium to obtain a second mixed solution, in which the cell preservation solution composition includes compound A and compound B, in composition of 100% of the cell preservation solution composition, the compound A ranges from 60.00wt% to 99.5wt% and the compound B ranges from 0.05wt% to 40.00wt%.

### BRIEF DESCRIPTION OF THE DRAWINGS

Fig. 1 is a flow chart showing the steps of preservation using a cell preservation solution composition after cell(s) have been performed with expansion culture process in accordance with disclosed herein;
Fig. 2 a flow chart showing the steps of preservation using a cell preservation solution composition to preserve the cell(s) after taken out of the cell bank and thawed in accordance with disclosed herein; and
Fig. 3 is a schematic diagram showing the relative survival rate of cell(s) after cells preserved in a cell preservation solution composition of the present invention and a commercial preservation solution at 4°C for 144 hours in accordance with the disclosed herein.

### DETAILED DESCRIPTION OF THE PREFERRED EMBODIMENT

The present invention provides a cell preservation solution composition, excluding dimethyl sulfoxide (DMSO). The cell preservation solution composition includes copolymer containing compound A and compound B, in composition of 100wt% of the cell preservation solution composition, the compound A ranges from 60.00wt% to 99.5wt% and the compound B ranges from 0.05wt% to 40.00wt%. In the present invention, compound A and compound B are mixed to form a copolymer, in which the compound A is the main chain of the copolymer and compound B is a side chain which bonded to the compound A, the general structural formula is as shown in formula (1):

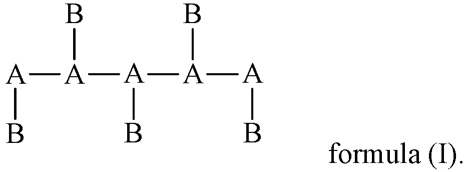

In another embodiment, compound A and compound B are straight-chain linkage, the general structural formula is as shown in formula (2), A-B formula (2). In above embodiments, compound A and compound B are mixed and is performed a polymerization process to form a copolymer.

In some preferred embodiments of the present invention, the compound A is polyethylene (PE), polyethersulfone (PES), polyethylene oxide (PEO), polydimethylsiloxane (PDMS), poly(methyl methacrylate) (PMMA), polyvinyl alcohol (PVA), polypropylene pyrrolidone (PPP), polyvinylpyrrolidone (PVP), polypropylene (PP), polyvinyl acetate (PVAc) or polyurethane (PU) and the compound B is polyacrylic acid (PAA), polyethylene glycol (PEG), polyvinylpyrrolidone (PVP), polypropylene glycol (PPG), methyl polyethylene glycol (mPEG) or polymethacrylic acid (PMA). The compound B is polyacrylic acid (PAA), polyethylene glycol (PEG), polyvinylpyrrolidone (PVP), polypropylene glycol (PPG), methyl polyethylene glycol (mPEG) or polymethacrylic acid (PMA).

In one embodiment of the present invention, the cell preservation solution composition further includes an additive, the objective of the additive is to reduce the damage to cell(s) and tissues during hypothermic preservation, in which the additive is 6-hydroxy-2, 5, 7, 8-tetramethylchroman-2-carboxylic acid, ammonium chloride, arginine, ascorbic acid, asparagine, boric acid, calcium acetate, calcium alginate, calcium lactate, calcium phosphate, dibasic dihydrate, calcium phosphate, dibasic anhydrous, glycine, histidine, leucine, magnesium carbonate, magnesium sulfate, malic acid, potassium alginate, potassium bicarbonate, potassium chloride, potassium citrate, potassium hydroxide, potassium metabisulfite, potassium metaphosphate, potassium nitrate, potassium phosphate, dibasic, proline, sodium acetate, sodium alginate, sodium bicarbonate, sodium chloride, sodium citrate dihydrate, sodium hyaluronate, sodium hydroxide, sodium lactate, sodium metabisulfite, sodium phosphate, dibasic, sodium phosphate, or monobasic.

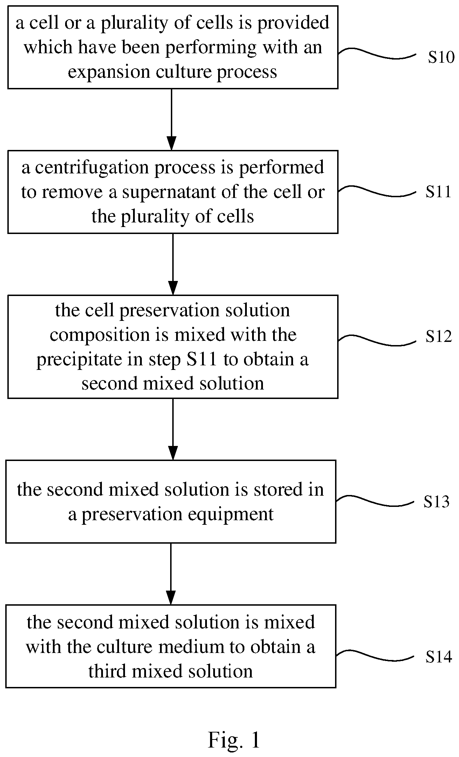

Please refer to Fig 1. Fig 1 is a flow chart showing the steps of hypothermic preservation using cell preservation solution composition after cell have been performed with expansion culture process according to the technology disclosed in this present invention. In Fig. 1, step S10: a cell or a plurality of cells is provided which have been performing with an expansion culture process. Step S11: a centrifugation process is performed to remove a supernatant of the cell or the plurality of cells. In this step, the cell or the plurality of cells is mixed with a media material to obtain a first mixed solution, the purpose of the media material is to enlarge the density range of the entire first mixed solution and to make the first mixed solution with a lower viscosity. The characteristics of the medium material must be less damaging to the cell structural components of the cell(s), easy to remove after the centrifugation process, and easy to determine the concentration of first mixed solution. The commonly used medium material includes hydrophilic organic molecules such as sucrose and glycerol, and heavy metal salts such as cesium chloride and cesium sulfate. It should be noted that user can choose the appropriate centrifugation method depend on the research object. The selection is based on the size and density of the particles and the characteristics of various centrifugation methods. In some embodiments of the present invention, the centrifugation method is a differential speed centrifugation or isopycnic centrifugation.

Take the differential speed centrifugation method as an example, the centrifugation process is performed to the first mixed solution at a first centrifugal speed to precipitate large-sized particle cell components of the first mixed solution, and to obtain the supernatant (or first supernatant). Next, if necessary, the first supernatant obtained in the first centrifugation step is subjected to perform a second centrifugal speed to precipitate cells with medium-sized particles and to obtain the supernatant (or second supernatant). After above centrifugation steps are completed, the second supernatant is removed to retain the precipitate.

Next, step S12: the cell preservation solution composition is mixed with the precipitate in step S11 to obtain a second mixed solution. In this step, the precipitates in step S11 are uniformly mixed with the cell preservation solution composition disclosed in the present invention under a temperature condition of 2°C-37°C, and after centrifugation, each centrifuge tube contains 100µL cells(100µL/Eppendorf) equivalent to 1×10⁵-1×10⁹ cells/mL. In this embodiment, take the automated cell counter as an example, by using image-based cell counters, or image cytometers, utilize advanced digital cameras, microscopes, and machine learning algorithms to count cells. The cell sample such as second mixed solution is first placed on a slide or cassette and loaded into the image cytometer, which takes multiple images and analyzes them. The automated cell counter then provides cell counts, viability, and size according to the multiple images and analyze results. It should be noted that cells must be stained for visualization, using methods like trypan blue staining. Therefore, we can obtain that the number of cells in the cell sample is 100µL cells(100µL/Eppendorf) equivalent to 1×10⁵-1×10⁹ cells/mL. Accordingly, the ability to see cells via the instrument's software enhances confidence in the machine learning algorithm. Overall, image cytometers improve cell counting efficiency by reducing errors caused by manual interpretation.

Step S13: the second mixed solution is stored in a preservation equipment. In this step, the preservation temperature range of the second mixed solution is 2°C-8°C, and the desired preservation time is set according to the user's needs, which can be set t=24*N, where N is positive integer. When the second mixed solution reaches the desired preservation time, the cells in the second mixed solution will be suspended, precipitated at the bottom of the centrifuge tube, or attached to the inner wall of the centrifuge tube. In this embodiment, the preservation equipment is refrigerator.

Next, step S14: the second mixed solution is mixed with the culture medium to obtain a third mixed solution. In this step, although the second mixed solution that has reached the desired preservation time in above step S13, however, the cell(s) of the second mixed solution will be suspended, precipitated at the bottom of the centrifuge tube or attached to the inner wall of the centrifuge tube, so the second mixed solution must be mixed evenly again, and then mixed with the culture medium to obtain the third mixed solution (mixture). In some embodiments, the cell(s) of second mixed solution is suspended, precipitated at the bottom of the centrifuge tube or attached to the inner wall of the centrifuge tube is remixed evenly with the second mixed solution, and then the second mixed solution with a volume of 10µL is taken to evenly mix with a culture medium with a volume of 1mL to obtain the third mixed solution. This third mixed solution is used for subsequent physiological and pathological research. In some embodiments, the culture medium is a medium containing DMEM (Dulbecco's Modified Eagle Medium), α-MEM (alpha-Minimum Essential Medium), bFGF (basic Fibroblast Growth Factor), EGF (Epidermal Growth Factor), HGF (Hepatocyte Growth Factor), SCF (Stem Cell Factor), glutathione, ascorbic acid, arginine, histidine, isoleucine, leucine, lysine, methionine, phenylalanine, proline, threonine, tryptophan, valine, cysteine, aspartic acid, glutamic acid, asparagine, glutamine, pyruvate, Vitamin A, Vitamin C, Vitamin D, Vitamin E, Vitamin K, riboflavin, thiamine, riboflavin, niacin, pantothenic acid, pyridoxine, folate, cobalamin, IGF (insulin growth factor), insulin-like growth factor, RPMI 1640 (Roswell Park Memorial Institute 1640), FBS (fetal bovine serum), fetal bovine plasma, human serum, IMDM (Iscove's Modified Dulbecco's Medium), Opti-MEM^{®} I Reduced Serum Medium, Hyclone^{™} , AdvanceSTEM^{™}, IL-2, X-VIVO^{™}, or Penicillin-Streptomycin.

In addition, in order to confirm the cell survival rate of using the cell preservation solution composition of the present invention as a cell preservation solution, the second mixed solution with a volume of 200pL is taken to place into a 96-well plate to analyze the cell viability level (or cell activity). In this invention, the determination step for the cell viability (or cell activity) is to take the aforementioned second mixed solution with a volume of 12.5µE to mix with the viability indicator with a volume of 12.5µE for staining. Next, the stained cell(s) is injected into a cell counting slide to calculate the number of cell(s), in which an automatic cell counter is used to calculate the number of the cell(s) after expansion culture process and do not need to be stored under the low temperature environment.

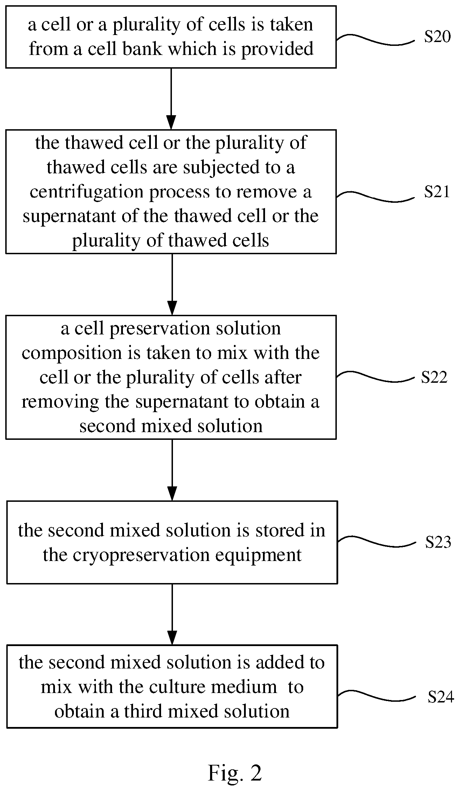

Please refer to Fig. 2. Fig. 2 is a flow chart showing the steps of preservation using a cell preservation solution composition to preserve the cell(s) after taken out of the cell bank and thawed. In Fig. 2, step S20: a cell or a plurality of cells is taken from a cell bank which is provided. In this step, the cell or the plurality of cells is in a hypothermic preservation condition, and a thawing process is performed to the hypothermic preservation cell or the plurality of hypothermic preservation cells to obtain a thawed cell or a plurality of thawed cells, so that the thawed cell or the plurality of thawed cells are then stored in the cell bank. Step S21: the thawed cell or the plurality of thawed cells are subjected to a centrifugation process to remove a supernatant of the thawed cell or the plurality of thawed cells. In this step, the thawed cell or the plurality of thawed cells is mixed with a media material to obtain a first mixed solution. Similarly, the purpose of the media material is to enlarge the density range of the entire first mixed solution and to make the first mixed solution with a lower viscosity. The characteristics of the medium material must be less damaging to the cell structural components of the cell(s), easy to remove after the centrifugation process, and easy to determine the concentration of first mixed solution. The commonly used medium material includes hydrophilic organic molecules such as sucrose and glycerol, and heavy metal salts such as cesium chloride and cesium sulfate. Then, user can choose the appropriate centrifugation method depend on the research object. The selection is based on the size and density of the particles and the characteristics of various centrifugation methods. In some embodiments of the present invention, the centrifugation method is a differential speed centrifugation or isopycnic centrifugation. In this embodiment, the centrifugation method is same as the aforementioned, so it is not to be repeatedly described herein.

Next, step S22: a cell preservation solution composition is taken to mix with the cell or the plurality of cells after removing the supernatant to obtain a second mixed solution. In this step, the cell or the plurality of cells after removing the supernatant is uniformly mixed with the cell preservation solution composition disclosed in this invention under a temperature range of 2°C-37°C to obtain the second mixed solution. It needs to be illustrated that the cell or the plurality of cells after removing the supernatant in each centrifuge tube includes 100µL cells (100µL/Eppendorf) (equivalent to 1×10⁵-1×10⁹ cells/mL).

Step S23: the second mixed solution is stored in the hypothermic preservation equipment. In this step, the preservation temperature of the second mixed solution is about 2°C-8°C, and the desired preservation time is set according to the user's requirement, in which the desired preservation time is t=24*N, where N is positive integer. In addition, during the hypothermic preservation process, the cell or the plurality of cells in the second mixed solution will be suspended, precipitated at the bottom of the centrifuge tube, or attached to the inner wall of the centrifuge tube when the second mixed solution reaches the target preservation time.

Next, step S24: the second mixed solution is added to mix with the culture medium to obtain a third mixed solution. Similarly, in this step, when the second mixed solution is performed with hypothermic preservation and stored in the hypothermic preservation equipment, the cell or the plurality of cells will be suspended, precipitated at the bottom of the centrifuge tube or attached to the inner wall of the centrifuge tube, so that after second mixed solution has reaches the desired preservation time in step S23 which must be mixed evenly again to mix with the culture medium to obtain the third mixed solution. In some embodiments, the cell or the plurality of cells of the second mixed solution may be suspended, precipitated at the bottom of the centrifuge tube or attached to the inner wall of the centrifuge tube which is remixed evenly with the second mixed solution, and then the second mixed solution with a volume of 10µL is taken to evenly mix with a culture medium with a volume of 1mL to obtain the third mixed solution. This third mixed solution is used for subsequent physiological and pathological research. In some embodiments, the culture medium is a medium containing DMEM(Dulbecco's Modified Eagle Medium), α-MEM (alpha-Minimum Essential Medium), bFGF(basic Fibroblast Growth Factor), EGF(Epidermal Growth Factor), HGF(Hepatocyte Growth Factor), SCF (Stem Cell Factor), glutathione, ascorbic acid, arginine, histidine, isoleucine, leucine, lysine, methionine, phenylalanine, proline, threonine, tryptophan, valine, cysteine, aspartic acid, glutamic acid, asparagine, glutamine, pyruvate, vitamin A, vitamin C, vitamin D, vitamin E, vitamin K, riboflavin, thiamine, niacin, pantothenic acid, pyridoxine, folate, cobalamin, IGF (insulin growth factor), insulin-like growth factor, RPMI 1640 (Roswell Park Memorial Institute 1640), FBS (Fetal Bovine Serum), fetal bovine plasma, human serum, IMDM (Iscove's Modified Dulbecco's Medium), Opti-MEM^{®} I Reduced Serum Medium, Hyclone^{™} , AdvanceSTEM^{™}, IL-2, X-VIVO^{™}, or Penicillin- Streptomycin.

As the described aforementioned, in order to confirm the cell survival rate of using the cell preservation solution composition of the present invention as a cell preservation solution, the second mixed solution with a volume of 200µLis taken to place into a 96-well plate to analyze the cell viability level (or cell activity). In this invention, the determination step for the cell viability (or cell activity) is to take the aforementioned second mixed solution with a volume of 12.5µL to mix with the viability indicator with a volume of 12.5µL for staining. Next, the stained cell(s) is injected into a cell counting slide to calculate the number of cell(s), in which an automatic cell counter is used to calculate the number of the cell(s) after expansion culture process and do not need to be stored under the low temperature.

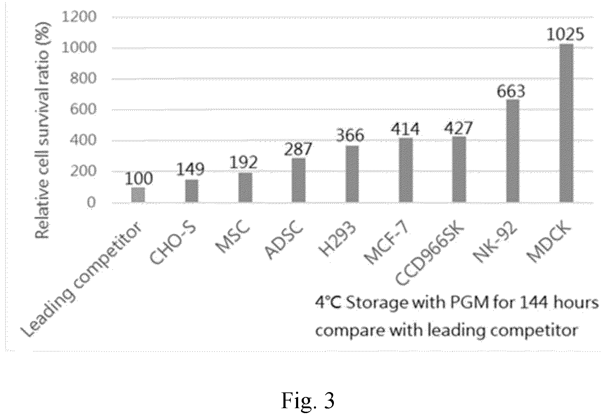

Next, please refer to Fig. 3. Fig. 3 is a schematic diagram showing the relative survival rate of cell(s) after cells preserved in a cell preservation solution composition of the present invention and a commercial preservation solution at 4°C for 144 hours. In Fig. 3, after the commercial preservation solution (leading competitor) (or control group) and the cell preservation solution composition of the present invention were preserved the cell under the same preservation conditions, for example, the temperature was 4°C and the preservation time was 144 hours, the relative survival rate is 100 for the commercial preservation solution, the cell relative survival rate of different cell is preserved by the cell preservation solution composition are listed as follows: the cell relative survival rate of CHO-S is 149, the cell relative survival rate of MSC is 192, the cell relative survival rate of ADSC is 287, the cell relative survival rate of H293 is 366, the cell relative survival rate of CCD966SK is 427, the cell relative survival rate of NK-92 is 663 and the cell relative survival rate of MDCK is 1025. As can be obtained from Fig. 3, the cell preservation solution composition of the present invention can serve as the cell preservation solution which can directly mix with the cell(s) under the temperature range is 2°C-37°C, so that the cell preservation solution composition of the present invention does not operate in a specific preservation environment. That is, the cell preservation solution composition of the present invention is not sensitive to the temperature, especially in the temperature range is 2°C-37°C. In addition, when the cell(s) is preserved by the cell preservation solution composition at a low temperature such as 2°C-8°C, the user can take it out and directly thaw and warm it up to 37°C before processing with subsequent steps. During the thawing process of the second mixture, the intensity of the cell preservation solution composition of the present invention will not damage the cell(s), allowing the cells to have a higher cell survival rate and the functionality of the cell(s) will not be lost due to the warming procedure of the cell preservation solution composition.

While the invention has been described in terms of what is presently considered to be the most practical and preferred embodiments, it is to be understood that the invention needs not be limited to the disclosed embodiments. On the contrary, it is intended to cover various modifications and similar arrangements included within the spirit and scope of the appended claims which are to be accorded with the broadest interpretation so as to encompass all such modifications and similar structures.

## Claims

1. A cell preservation solution composition, which excludes dimethyl sulfoxide, and comprises a copolymer containing compound A and compound B, in a composition of 100wt% of the cell preservation solution composition, the compound A ranges from 60.00wt% to 99.5wt% and the compound B ranges from 0.05wt% to 40.00wt%.

2. The cell preservation solution composition according to claim 1, wherein the compound A is polyethylene (PE), polyethersulfone (PES), polyethylene oxide (PEO), polydimethylsiloxane (PDMS), poly(methyl methacrylate) (PMMA), polyvinyl alcohol (PVA), polypropylene pyrrolidone (PPP), polyvinylpyrrolidone (PVP), polypropylene (PP), polyvinyl acetate (PVAc) or polyurethane (PU) and the compound B is polyacrylic acid (PAA), polyethylene glycol (PEG), polyvinylpyrrolidone (PVP), polypropylene glycol (PPG), methyl polyethylene glycol (mPEG) or polymethacrylic acid (PMA).

3. The cell preservation solution composition according to claim 1, wherein the compound A used as the main chain of the copolymer, and the compound B is a side chain which bonded to the compound A.

4. The cell preservation solution composition according to claim 1, wherein the compound A is bonded the compound B are straight-chain linkage.

5. The cell preservation solution composition according to claim 1, further comprising an additive, wherein the additive is 6-hydroxy-2, 5, 7, 8-tetramethylchroman-2-carboxylic acid, ammonium chloride, arginine, ascorbic acid, asparagine, boric acid, calcium acetate, calcium alginate, calcium lactate, calcium phosphate, dibasic dihydrate, calcium phosphate, dibasic anhydrous, glycine, histidine, leucine, magnesium carbonate, magnesium sulfate, malic acid, potassium alginate, potassium bicarbonate, potassium chloride, potassium citrate, potassium hydroxide, potassium metabisulfite, potassium metaphosphate, potassium nitrate, potassium phosphate, dibasic, proline, sodium acetate, sodium alginate, sodium bicarbonate, sodium chloride, sodium citrate dihydrate, sodium hyaluronate, sodium hydroxide, sodium lactate, sodium metabisulfite, sodium phosphate, dibasic, sodium phosphate or monobasic.

6. A method for hypothermic preservation of cell using a cell preservation solution composition as described in claim 1, includes providing a cell or a plurality of cells have been performing with an expansion culture process, performing a centrifugation process to remove a supernatant of the cell or the plurality of cells to remove a supernatant of the cell or the plurality of cells, the **characterized in that**:
mixing the cell or the plurality of cells which the supernatant has been removed with a cell preservation solution composition to obtain a first mixed solution;
performing a hypothermic preservation process on the first mixed solution until a desired preservation time; and
mixing the first mixed solution that has reached the desired preservation time with a culture medium to obtain a second mixed solution,
wherein the cell preservation solution composition includes compound A and compound B, in a composition of 100% of the cell preservation solution composition, the compound A ranges from 60.00wt% to 99.5wt% and the compound B ranges from 0.05wt% to 40.00wt%.

7. The method according to claim 6, wherein the temperature range of hypothermic preserving the first mixture is 2°C-8°C.

8. The method according to claim 6, wherein the culture medium is a medium containing DMEM (Dulbecco's Modified Eagle Medium), α-MEM (alpha-Minimum Essential Medium), bFGF (basic fibroblast growth factor), EGF (epidermal growth factor), HGF (hepatocyte growth factor), SCF (stem cell factor), glutathione, ascorbic acid, arginine, histidine, isoleucine, leucine, lysine, methionine, phenylalanine, proline, threonine, tryptophan, valine, cysteine, aspartic acid, glutamic acid, asparagine, glutamine, pyruvate, vitamin A, vitamin C, vitamin D, vitamin E, vitamin K, riboflavin, thiamine, niacin, pantothenic acid, pyridoxine, folate, cobalamin, IGF (insulin growth factor), insulin-like growth factor, RPMI 1640(Roswell Park Memorial Institute 1640), FBS (Fetal Bovine Serum), fetal bovine plasma, human serum , IMDM (Iscove's Modified Dulbecco's Medium), Opti-MEM^{®} I Reduced Serum Medium, Hyclone^{™}, AdvanceSTEM^{™}, IL-2, X-VIVO^{™}, or penicillin-streptomycin.

9. The method according to claim 6, wherein the target preservation time is 24*N, wherein N is a positive integer.

10. A method for hypothermic preservation of cell using a cell preservation solution composition as described in claim 1, includes providing a cell or a plurality of cells is taken from a cell bank, performing a centrifugation process to remove a supernatant of the cell or the plurality of cells, the **characterized in that**:
mixing the cell or the plurality of cells after removing the supernatant with a cell preservation solution composition to obtain a first mixed solution;
performing a hypothermic preservation process to the first mixed solution until a desired preservation time; and
mixing the first mixed solution that has reached the desired preservation time with a culture medium to obtain a second mixed solution,
wherein the cell preservation solution composition includes compound A and compound B, in composition of 100% of the cell preservation solution composition, the compound A ranges from 60.00wt% to 99.5wt% and the compound B ranges from 0.05wt% to 40.00wt%.

11. The method according to claim 10, wherein the cell or the plurality of cells is a thawed cell or a plurality of thawed cells that is stored in the cell bank.

12. The method according to claim 10, wherein the temperature range of hypothermic preservation process for hypothermic preserving the first mixed solution is 2°C-8°C.

13. The method according to claim 10, wherein the culture medium is DMEM (Dulbecco's Modified Eagle Medium), α-MEM (alpha-Minimum Essential Medium), bFGF (basic fibroblast growth factor), EGF (epidermal growth factor), HGF (hepatocyte growth factor), SCF (stem cell factor), glutathione, ascorbic acid, arginine, histidine, isoleucine, leucine, lysine, methionine, phenylalanine, proline, threonine, tryptophan, valine, cysteine, aspartic acid, glutamic acid, asparagine, glutamine, pyruvate, vitamin A, vitamin C, vitamin D, vitamin E, vitamin K, riboflavin, thiamine, niacin, pantothenic acid, pyridoxine, folate, cobalamin, IGF (insulin growth factor), insulin-like growth factor, RPMI 1640(Roswell Park Memorial Institute 1640), FBS (Fetal Bovine Serum), fetal bovine plasma, human serum IMDM (Iscove's Modified Dulbecco's Medium), Opti-MEM^{®} I Reduced Serum Medium, Hyclone^{™}, AdvanceSTEM^{™}, IL-2, X-VIVO^{™}, or penicillin-streptomycin.

14. The method according to claim 10, wherein the target preservation time is 24*N, wherein N is a positive integer.

## Amended claims

### Amended claims in accordance with Rule 137(2) EPC.

1. A cell preservation solution composition, which excludes dimethyl sulfoxide, and comprises a copolymer containing compound A and compound B, in a composition of 100wt% of the cell preservation solution composition, the compound A ranges from 60.00wt% to 99.5wt% and the compound B ranges from 0.05wt% to 40.00wt%, wherein the compound A is polyvinyl alcohol (PVA) and the compound B is polyethylene glycol (PEG), the compound A forms a straight main chain, and the compound B serves as a side chain bonded to the compound A.

2. The cell preservation solution composition according to claim 1, further comprising an additive, wherein the additive is 6-hydroxy-2, 5, 7, 8-tetramethylchroman-2-carboxylic acid, ammonium chloride, arginine, ascorbic acid, asparagine, boric acid, calcium acetate, calcium alginate, calcium lactate, calcium phosphate, dibasic dihydrate, calcium phosphate, dibasic anhydrous, glycine, histidine, leucine, magnesium carbonate, magnesium sulfate, malic acid, potassium alginate, potassium bicarbonate, potassium chloride, potassium citrate, potassium hydroxide, potassium metabisulfite, potassium metaphosphate, potassium nitrate, potassium phosphate, dibasic, proline, sodium acetate, sodium alginate, sodium bicarbonate, sodium chloride, sodium citrate dihydrate, sodium hyaluronate, sodium hydroxide, sodium lactate, sodium metabisulfite, sodium phosphate, dibasic, sodium phosphate or monobasic.

3. A method for hypothermic preservation of cell using a cell preservation solution composition as described in claim 1, the method including providing a cell or a plurality of cells which have been performed with an expansion culture process, performing a centrifugation process to remove a supernatant of the cell or the plurality of cells, **characterized in that** the method includes:
mixing the cell or the plurality of cells, of which the supernatant has been removed, with a cell preservation solution composition to obtain a first mixed solution;
performing a hypothermic preservation process on the first mixed solution until a desired preservation time; and
mixing the first mixed solution that has reached the desired preservation time with a culture medium to obtain a second mixed solution,
wherein the cell preservation solution composition includes compound A and compound B, in a composition of 100% of the cell preservation solution composition, the compound A ranges from 60.00wt% to 99.5wt% and the compound B ranges from 0.05wt% to 40.00wt%, wherein the compound A is polyvinyl alcohol (PVA) and the compound B is polyethylene glycol (PEG), the compound A forms a straight main chain, and the compound B serves as a side chain bonded to the compound A.

4. The method according to claim 3, wherein the temperature range of hypothermic preserving the first mixture is 2°C-8°C.

5. The method according to claim 3, wherein the culture medium is a medium containing DMEM (Dulbecco's Modified Eagle Medium), α-MEM (alpha-Minimum Essential Medium), bFGF (basic fibroblast growth factor), EGF (epidermal growth factor), HGF (hepatocyte growth factor), SCF (stem cell factor), glutathione, ascorbic acid, arginine, histidine, isoleucine, leucine, lysine, methionine, phenylalanine, proline, threonine, tryptophan, valine, cysteine, aspartic acid, glutamic acid, asparagine, glutamine, pyruvate, vitamin A, vitamin C, vitamin D, vitamin E, vitamin K, riboflavin, thiamine, niacin, pantothenic acid, pyridoxine, folate, cobalamin, IGF (insulin growth factor), insulin-like growth factor, RPMI 1640(Roswell Park Memorial Institute 1640), FBS (Fetal Bovine Serum), fetal bovine plasma, human serum, IMDM (Iscove's Modified Dulbecco's Medium), Opti-MEM^{®} I Reduced Serum Medium, Hyclone^{™}, AdvanceSTEM^{™}, IL-2, X-VIVO^{™}, or penicillin-streptomycin.

6. The method according to claim 3, wherein the target preservation time is 24*N, wherein N is a positive integer.

7. A method for hypothermic preservation of cell using a cell preservation solution composition as described in claim 1, the method including providing a cell or a plurality of cells taken from a cell bank, performing a centrifugation process to remove a supernatant of the cell or the plurality of cells, **characterized in that** the method includes:
mixing the cell or the plurality of cells with a cell preservation solution composition after removing the supernatant to obtain a first mixed solution;
performing a hypothermic preservation process on the first mixed solution until a desired preservation time; and
mixing the first mixed solution that has reached the desired preservation time with a culture medium to obtain a second mixed solution,
wherein the cell preservation solution composition includes compound A and compound B, in composition of 100% of the cell preservation solution composition, the compound A ranges from 60.00wt% to 99.5wt% and the compound B ranges from 0.05wt% to 40.00wt%, wherein the compound A is polyvinyl alcohol (PVA) and the compound B is polyethylene glycol (PEG), the compound A forms a straight main chain, and the compound B serves as a side chain bonded to the compound A.

8. The method according to claim 7, wherein the cell or the plurality of cells is a thawed cell or a plurality of thawed cells that is stored in the cell bank.

9. The method according to claim 7, wherein the temperature range of hypothermic preservation process for hypothermic preserving the first mixed solution is 2°C-8°C.

10. The method according to claim 7, wherein the culture medium is DMEM (Dulbecco's Modified Eagle Medium), α-MEM (alpha-Minimum Essential Medium), bFGF (basic fibroblast growth factor), EGF (epidermal growth factor), HGF (hepatocyte growth factor), SCF (stem cell factor), glutathione, ascorbic acid, arginine, histidine, isoleucine, leucine, lysine, methionine, phenylalanine, proline, threonine, tryptophan, valine, cysteine, aspartic acid, glutamic acid, asparagine, glutamine, pyruvate, vitamin A, vitamin C, vitamin D, vitamin E, vitamin K, riboflavin, thiamine, niacin, pantothenic acid, pyridoxine, folate, cobalamin, IGF (insulin growth factor), insulin-like growth factor, RPMI 1640(Roswell Park Memorial Institute 1640), FBS (Fetal Bovine Serum), fetal bovine plasma, human serum, IMDM (Iscove's Modified Dulbecco's Medium), Opti-MEM^{®} I Reduced Serum Medium, Hyclone^{™}, AdvanceSTEM^{™}, IL-2, X-VIVO^{™}, or penicillin-streptomycin.

11. The method according to claim 7, wherein the target preservation time is 24*N, wherein N is a positive integer.
